# EUROPEAN PATENT APPLICATION

(11) **EP 4 552 685 A1**
(43) Date of publication of application: **14.05.2025**
(21) Application number: 23208150.5
(22) Date of filing: 07.11.2023
(51) Int. Cl.: A61N 1/375, A61N 1/362, A61N 1/39

(54) **MEDICAL DEVICE, PARTICULARLY IMPLANT, COMPRISING A HEADER GUIDE**

(71) Applicant: BIOTRONIK SE & Co. KG, 12359 Berlin (DE)
(72) Inventor: Kolberg, Gernot, 12161 Berlin (DE); Fariborz, Armin, 14193 Berlin (DE); Luettke, Vivien, 10553 Berlin (DE)
(74) Representative: Biotronik Corporate Services SE

(57) **Abstract**

The present invention relates to a medical device (1), comprising a header (2) having an outer surface (2a), wherein the header (2) comprises a configured to receive a first electrical connector socket for receiving a first plug (30) of a first electrode lead in an axial direction (x) of the first electrical connector socket (3), and a first distal section (2) being formed in the outer surface (2a), the first distal (20) comprising an inner surface (21) and leading to the first proximal section . According to the invention, the inner surface (21) comprises a rounding (22) in at least one plane comprising said axial direction (x).

## Description

The present invention relates to a medical device.

The shapes of headers of implantable pacemakers and defibrillators are often designed in such a way that an electrode lead exits a header bore at an undesired angle. Often, the lead exits the header laterally, making it easy for the user to guide the lead along the side of the casing for a relieved exit. This also allows to arrange an excess length of the electrode lead to be suitably arranged during implantation.

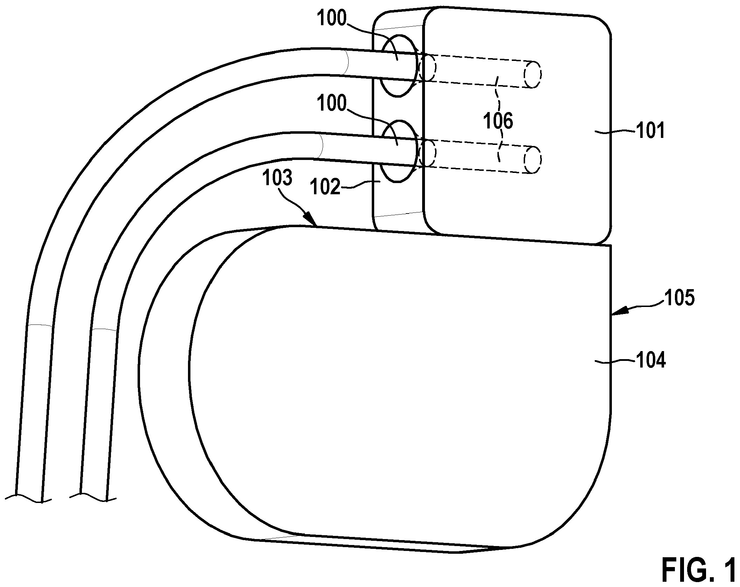

To facilitate unloaded electrode lead guidance, as shown in Fig. 1, the electrode lead 100 must not only exit the header 101 perpendicular to the header surface 102, it must also run a distance parallel to the long edge 103 of the housing 104. Ideally, then, the header 101 sits on an end of the long edge 103 of the device's housing 104 and is typically flush with a housing wall 105 opposite to the header surface 102, which excludes header holes that run perpendicular to the long edge 103 or whose exit terminates with casing wall 105. However, it is precisely such an arrangement that allows a compact device design suitable for production, which is currently only conceivable to a limited extent.

Therefore, the problem to be solved by the present invention is to provide a medical device that reduces the risk of exposing an electrode lead to breakage by bending.

This problem is the solved by a medical device according to the feature of claim 1. Preferred embodiments of this aspect of the present invention are stated in the dependent claims and are described below.

According to claim 1, a medical device is disclosed, the medical device comprising a header having an outer surface, wherein the header comprises a first throughgoing header bore with
- a first proximal section configured to receive first electrical connector socket for receiving a first plug of a first electrode lead in an axial direction of the first proximal section, and
- a first distal section formed in the outer surface, wherein the first distal section comprises an inner surface that defines the distal section, and wherein the first distal leads to the first proximal to provide access to the first electrical connector socket so as to allow the first plug to be plugged into the first electrical connector socket through said first distal section in the axial direction.

According to the invention, the inner surface comprises a preferably convex rounding in at least one plane comprising said axial direction particularly so as to limit a bending of the first electrode lead connected to the first plug, when the first plug is plugged into the first electrical connector socket and the electrode lead tightly contacts and follows the rounding of the first distal section.

Thus, due to the rounding it can be prevented that an electrode lead leaving the first through header bore, particularly the first distal section thereof, is bent sharply at the header edge if the electrode lead is not guided properly. At this sharp edge that is present in the prior art (cf. e.g. Fig. 1), a risk of breakage of the lead is present. The user must reduce this risk by positioning the lead during implantation in such a way that kinking is avoided. Due to the present invention such risk of breakage can be reduced or avoided and the user is less limited regarding arrangement of the lead. Of course, the invention may also be applied to several such leads leaving the header.

Particularly, the fact that the rounding lies in said afore-described plane means that a contour of the inner surface describing the rounding extends in said plane. In other words, said rounding extends in at least one angular orientation corresponding to an azimuth angle in a plane perpendicular to the axial direction of the plug / connector socket. According to an embodiment, said rounding extends towards the nearest housing wall that runs along said axial direction.

The invention is particularly suited for providing a bend relief for connectors according to one of the standards IS1, IS4, DF4, DF1. Particularly, the electrode lead is already restricted in the bending radius when it leaves the header in such a way that breakage at the header hole edge region can be reduced or ruled out. The housing of the device ma thus be made considerably smaller, because the respective lead does not have to be guided by the housing.

According to an embodiment of the invention, due to the convex rounding, a cross section of the header hole perpendicular to the axial direction increases - starting from the connector - towards a circumferential edge region of the header hole, via which edge region the inner surface of the header hole transitions into said outer surface of the header.

Furthermore, according to an embodiment of the invention, a smallest radius of the rounding (particularly in said plane) is larger than 0.5 mm, particularly equal to or larger than 2 mm.

Further, according to an embodiment of the invention, the inner surface of the header hole comprises a convex curved contour in the region of the rounding, which contour lies in said plane.

According to yet another embodiment of the present invention, the contour comprises a convex curvature having a radius being larger than 0.5 mm, particularly equal to or larger than 2 mm.

In an embodiment of the invention, the course of the contour or a section thereof is defined by a continuous mathematical function, wherein preferably the mathematical function is one of: a parabola, a hyperbola, a section of an ellipse, an exponential function.

Furthermore, in an embodiment of the invention, the inner surface of the first distal section of the first throughgoing header bore comprises an edge region extending in a circumferential direction of the first distal section, via which edge region the inner surface of the first distal section transitions into said outer surface of the header. Preferably, according to an embodiment, said rounding extends at least over a part of the edge region in the circumferential direction of the edge region, particularly at least over one of: 10% of the edge region, 20% of the edge region, 30% of the edge region, 40% of the edge region, 50% of the edge region, 60% of the edge region, 70% of the edge region, 80% of the edge region, 90% of the edge region, 100% of the edge region.

Furthermore, according to an embodiment of the invention, said contour changes its shape in the circumferential direction of said edge region.

According to yet another embodiment of the invention, the shape of the first header opening or bore conforms to a technical standard, particularly one of the following technical standards: IS1, IS4, DF4, DF1. Preferably, according to an embodiment, the rounding of the header hole lies outside the range defined by the technical standard.

Furthermore, in an embodiment of the invention, the medical device is an implantable medical device. Preferably, according to an embodiment, the implantable medical device is an implantable cardiac pacemaker or an implantable cardioverter defibrillator. According to an embodiment, the medical device comprises a first electrical connector socket being arranged in the first proximal section of the first throughgoing header bore. According to further embodiment of the invention, the medical device comprises first plug, the first plug being connected to a proximal end of a first electrode lead of the medical device, wherein the first electrode lead preferably comprises at least one electrode or multiple electrodes arranged in a distal end section of the electrode lead, wherein particularly the first plug is electrically connected to the first electrode connector socket. Furthermore, according to an embodiment of the invention, the medical device comprises a housing for accommodating an energy storage, e.g., a battery and/or one ore more capacitors, and an electronic module of the medical device, wherein the header is connected to the housing. Furthermore, according to yet another embodiment of the invention, the first electrical connector socket is electrically connected to the electronic module via an electrical feedthrough.

Particularly, the present invention may be applied to more than one electrical connector socket. Particularly, several electrical connector sockets, particularly two or three such connectors, may be equipped with header openings or bores comprising a rounding to avoid breakage of corresponding electrode leads.

For example, in an embodiment of the present invention, the header may comprises a second throughgoing header bore with
- a second proximal section configured to receive second electrical connector socket for receiving a second plug of a second electrode lead in an axial direction of the second proximal section, and
- a second distal section formed in the outer surface, the second distal section comprising an inner surface defining the second distal section and leading to the second proximal section, wherein the second distal section provides access to the second electrical connector socket so as to allow the second plug to be plugged into the second electrical connector socket through said second header opening or bore in the axial direction of the second proximal section.

Furthermore, preferably, the inner surface of the second distal section comprises a convex rounding in at least one plane comprising said axial direction of the second proximal section, particularly so as to limit a bending of a second electrode lead connected to the second plug, when the second plug is plugged into the second electrical connector socket, and the second electrode lead tightly contacts and follows the rounding of the inner surface of the second distal section. Preferably, the first second throughgoing header bore or bore are characterized by an identical rounding or shape of its distal sections.

In the following embodiments of the present invention as well as further features and advantages of the present invention shall be described with reference to the Figures, wherein
- Fig. 1: shows a medical device in form of a pacemaker according to the state of the art,
- Fig. 2: shows a header of an embodiment of a medical device according to the present invention,
- Fig. 3: shows a header of a further embodiment of a medical device according to the present invention,
- Fig. 4: shows a header of a further embodiment of a medical device according to the present invention, and
- Fig. 5: shows an embodiment of a medical device according to the present invention comprising the header according to the embodiment shown in Fig. 2 as an example.

Fig. 1 schematically shows a header 101 with conventional header bores 106 configured to receive electrical connector socket for receiving electrode leads, particularly in a proximal section of the bores 106. Particularly, the exit / edge (distal) region of the respective header bore 106 is radially sharp-edged all around. The typical edge radii are 0.2mm. The standard also requires a test in which the electrode must be bent by an edge radius of 0.2 mm. The user must ensure that the electrode lead 100 is not bent (kinked) 90° immediately after the respective exit. Usually, therefore, the housing 104 continues in the direction of the electrode lead(s) 100. Then, it is easy for the user to also lay the electrode lead in the direction of the housing 104. A casing design in which the casing 104 terminates with the front side 102 of the header 101 is regulatorily not permitted.

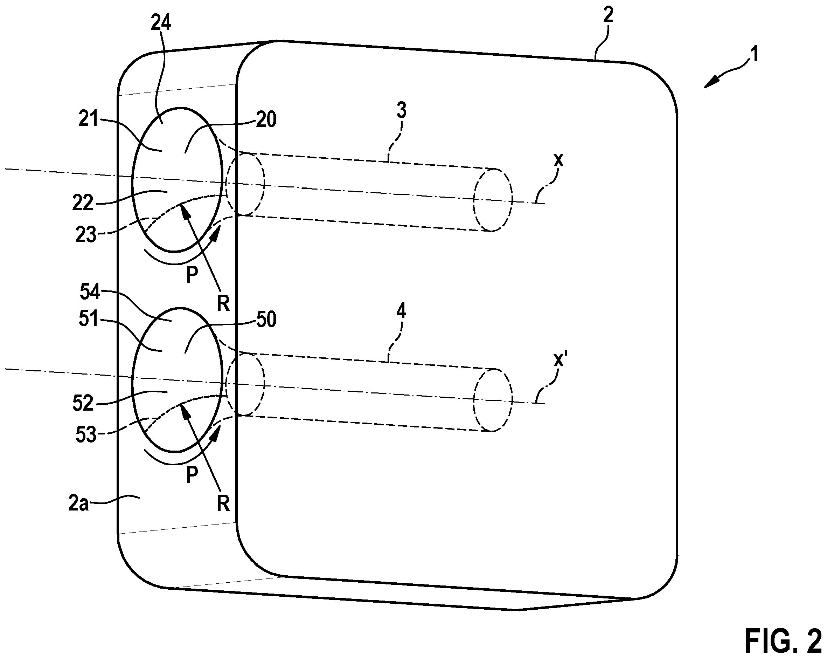

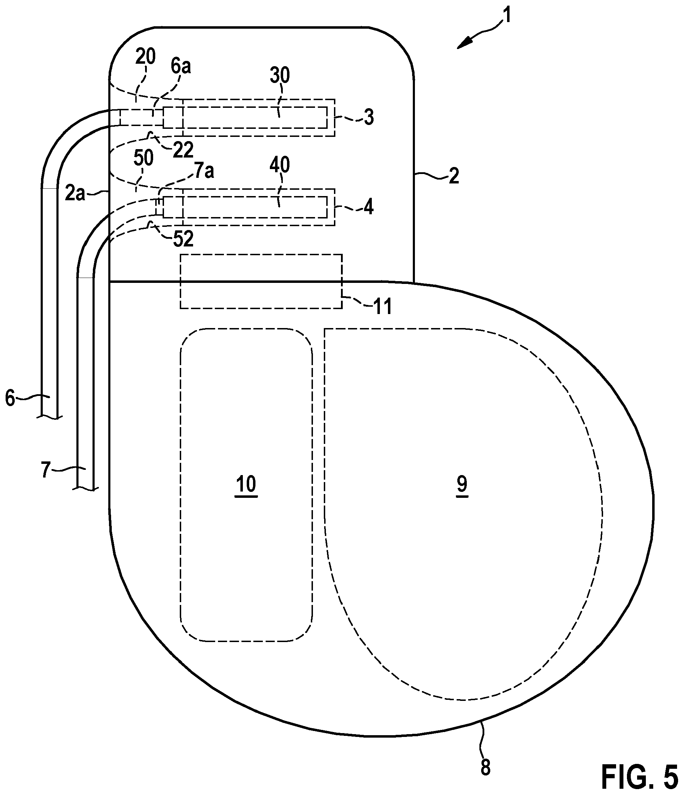

Fig. 2 now shows an embodiment of a medical device 1 according to the present invention, comprising a header 2 having an outer surface 2a, wherein the header 2 comprises a first header bore 3, 20. The first header bore 3, 20 comprise a first proximal section 3 configured to receive a first electrical connector socket 3 for receiving a first plug 30 of a first electrode lead (cf. Fig. 5) in an axial direction x of the first proximal section 3. The first header bore 3, 20 further comprises a first distal section 20 formed in the outer surface 2a, wherein the first distal section 20 comprises an inner surface 21 that defines the first distal section 20, and wherein the first distal section 20 further extends towards first proximal section 3 to provide access to the electrical connector socket being arranged in the proximal section 3 so as to allow the first plug 30 to be plugged into the first electrical connector socket 3 through the first distal section 20 in the axial direction x.

Particularly, the header 2 may comprise a second header bore 4, 50 with a second proximal section 4 configured to receive a second electrical connector socket 3 for receiving a second plug 40 of a second electrode lead 7 and a second distal section 50, wherein the second distal section 50 extends towards the second proximal section 50 to provide access to a second electrical connector socket being arranged in the second proximal section 3 so as to allow the second plug 40 to be plugged into the second electrical connector socket 3 through the second distal section 20 in the axial direction x. Particularly, the second distal section is designed as the first distal section 20.

In the following, the headers 2 are described as having two header bores 3,4, 20, 50, but the number of header bores may be adapted to the technical requirements of the respective device 1 considered. Preferably, the inner surface 21, 51 of the respective distal sections 20, 50 comprises a preferably convex rounding 22, 52. Particularly, the rounding 22 extends in at least one plane in which the axial direction x lies, and wherein the rounding 52 extends in at least one plane in which the axial direction x' lies. The respective rounding 22, 52 delimits a bending of an electrode lead 6, 7 connected to the respective plug 30, 40, when the respective plug 30, 40 is plugged into the associated electrical connector sockets arranged in the respective proximal sections 3, 4 and the lead 6, 7 tightly contacts and follows the respective rounding 22, 52 (cf. Fig. 5).

As shown in Fig. 2, a curvature of the respective rounding 22, 53 may be characterized by considering a contour 23, 53 of the respective inner surface 21, 51, which contour 23, 53 lies in the plane in which the respective axial directions x, x' extends, too.

In case of the embodiment of Fig. 2, the rounding 22, 52 extends over a complete edge region 24, 54 of the respective distal section 20, 50 in the respective circumferential direction P, i.e., the rounding 22, 52 / contour 23, 53 is symmetric with respect to a rotation (azimuth) around the respective axial direction x, x'.

Thus, the configuration of distal sections 20, 50 shown in Fig. 2 allows bending the respective electrode lead 6, 7 (cf. Fig. 5) by 90° immediately after exiting the header 2 without being subjected to a tight bending radius (kink). A bending radius R of the respective contour 23, 53 of 0.5 mm is already sufficient to protect the respective electrode lead 6, 7. Ideally, the bending radius R is about 2 mm or larger.

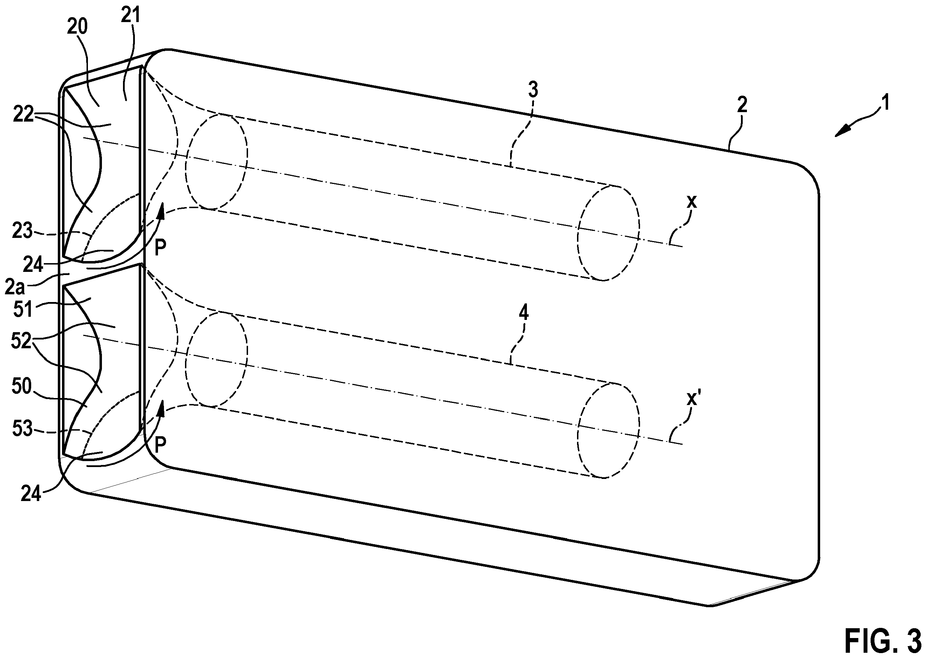

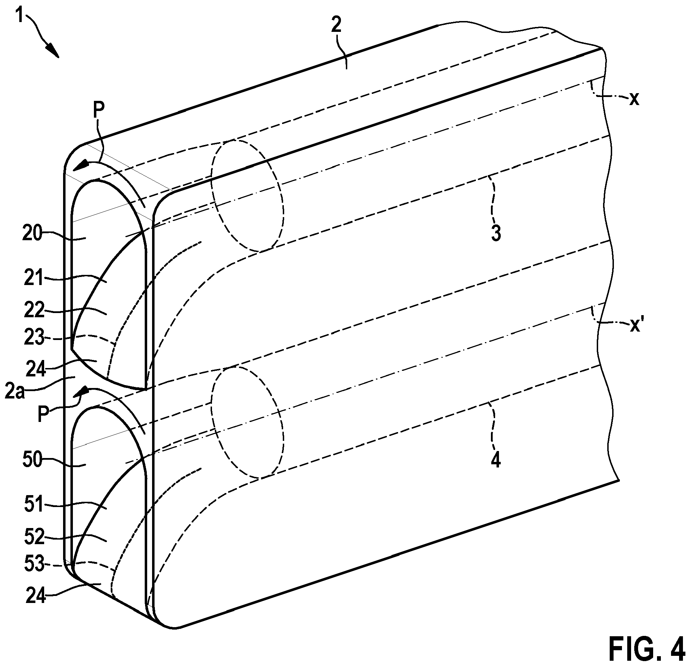

Depending on the direction in which the respective electrode lead 6, 7 is to be led out of the header 2, the bending radius may be differently dimensioned in different directions. For example, it may not be necessary for the edge region 24 to have a large radius of curvature for decoupling the electrode lead 6, 7 laterally, because lateral decoupling is less likely. The header 2 may therefore be kept narrow as shown in Figs. 3 and 4. Furthermore, also an upward bend of electrode leads is less likely so that an upward radius is also not necessary so that the header 2 may then be kept flat (cf. Fig. 4).

Particularly, as shown in Fig 3, the distal section 20, 50 comprise roundings 22, 52 on two opposing portions of the inner surface 21, 51 / boundary region 24, 53 of the respective distal section 20, 50, but not around the whole edge region 24 in the circumferential direction P as shown in Fig. 2. As a consequence, the contour 23, 53 changes its shape in the circumferential direction P along the edge region 24 of the respective distal section 20, 50 significantly.

Fig. 4 shows a section of a header 2 designed for the electrode leads to be bent downwards towards the housing 104 immediately after leaving the header 2. The header 2 is no more suitable than conventional headers for bends in other directions. I.e., here the respective rounding 22, 52 is only present on one side of the inner surface 21, 51 / edge region 24, 54 permitting to bend the respective electrode lead so as to conform to the indicated contour 23, 53 of the respective rounding 22, 52.

Fig. 5 shows a medical device 1 according to the present invention, here with a header 2 according to the embodiment of Fig. 2 (the embodiments of Figs. 3 and 4 may also be employed instead). Advantageously, the roundings 22, 52 of the distal section 20, 50 may permit one to bend the leads 6, 7 coming from the header 2 downwards by 90° without damaging them. This allows one to have the front side / outer surface 2a of the header 2 to be flush with the lateral surface of the housing 8 which achieves a compact design of the device 1 in contrast to Fig. 1

Particularly, the medical device 1 may be an implantable medical device 1 such as an implantable cardiac pacemaker 1 or an implantable cardioverter defibrillator 1. The medical device 1 can comprise plugs 30, 40 connected to a proximal end 6a, 7a of the respective electrode lead 6, 7 of the medical device 1, respectively, wherein the respective electrode lead 6, 7 may comprises at least one electrode or multiple electrodes arranged on a distal end section of the respective electrode lead 6, 7 (not shown). Furthermore, the medical device 1 may comprise a housing 8 for accommodating an energy storage 9, e.g., a battery 9, and an electronic circuit or electronic module 10 of the medical device 1, wherein the header 2 is particularly connected to the housing 8. Furthermore, particularly, the respective electrical connector 3, 4 is electrically connected to the electronic circuit 10 via an electrical feedthrough 11.

The solution according to the present invention advantageously expands the design options for medical devices such as pacemakers. The electrode leads may leave the device at the free side and be bent directly at the header. Very slim device designs may thus be created. A user would be irritated if expected to bend the electrode lead sharply around the edge of the bore. With a bore according to the invention, the bending direction would be intuitively given to the user and at the same time the electrode lead would be protected from too tight radii.

## Claims

1. A medical device (1), comprising a header (2) having an outer surface (2a), wherein the header (2) comprises a first header bore with
- a first proximal section (3) configured to receive a first electrical connector socket for receiving a first plug (30) of a first electrode lead in an axial direction (x) of the first proximal section (3), and
- a first distal section (20) being formed in the outer surface (2a), the first distal (20) comprising an inner surface (21) and leading to the first proximal section (3),
**characterized in that**
the inner surface (21) comprises a rounding (22) in at least one plane comprising said axial direction (x).

2. The medical device according to claim 1, wherein a smallest radius (R) of the rounding (22) is equal to or larger than 0.5 mm, particularly larger or equal to 2 mm.

3. The medical device according to claim 1 or 2, wherein the inner surface (21) of the first distal section (20) comprises a contour (23) in the region of the rounding (21), which contour (23) extends in said plane.

4. The medical device according to claim 3, wherein the contour (23) comprises a convex curvature having a radius (R) being equal to or larger than 0.5 mm, particularly larger or equal to 2 mm.

5. The medical device according to claim 3 or 4, wherein the contour (23) or a section thereof is defined by a continuous mathematical function, wherein preferably the mathematical function is one of: a parabola, a hyperbola, an ellipse, an exponential function.

6. The medical device according to one of the preceding claims, wherein the inner surface (21) of the first distal section (20) comprises an edge region (24) extending in a circumferential direction (P) of the first distal section (20), via which edge region (24) the inner surface (21) of the first distal section (20) transitions into said outer surface (2a) of the header (2).

7. The medical device according to claim 6, wherein said rounding (22) extends at least over a part of the edge region (24) in the circumferential direction of the edge region (P), particularly at least over one of: 10% of the edge region (24), 20% of the edge region (24), 30% of the edge region (24), 40% of the edge region (24), 50% of the edge region (24), 60% of the edge region (24), 70% of the edge region (24), 80% of the edge region (24), 90% of the edge region (24), 100% of the edge region (24).

8. The medical device according to claim 3 and according to claim 6 or 7, wherein said contour (23) changes its shape along the circumferential edge region (24).

9. The medical device according to one of the preceding claims, wherein the medical device (1) is an implantable medical device (1).

10. The medical device according to claim 9, wherein the implantable medical device (1) is an implantable cardiac pacemaker or an implantable cardioverter defibrillator.

11. The medical device according to one of the preceding claims, wherein the medical device (1) comprises
- a first electrical contact socket being arranged in the proximal section (3) of the first header bore (3, 20), and
- a first electrode lead with a first plug (30), the first plug (30) being connected to a proximal end (6a) of the first electrode lead (6) of the medical device (1), and the first plug (3) being further connected to the first electrical contact socket.

12. The medical device according to one of the preceding claims, wherein the medical device (1) comprises a housing (8) for accommodating an energy storage (9) and an electronic module (10) of the medical device (1), wherein the header (2) is connected to the housing (8).

13. The medical device according to claim 12, wherein the electrical connector socket is electrically connected to the electronic module (10) via an electrical feedthrough (11).

14. The medical device according to one of the preceding claims, wherein the header (2) comprises a second header bore (4, 50) with
- a second proximal section configured to receive a second electrical connector socket (4) for receiving a second plug (40) of a second electrode lead in an axial direction (x') of the second electrical connector socket (4), and
- a second distal section (50) formed in the outer surface (2a), the second distal section (50) comprising an inner surface (51) and leading to the second proximal section (4), and wherein the inner surface (51) of the second distal section (50) comprises a rounding (52) in at least one plane comprising said axial direction (x') of the second proximal section (4).
